(19) Europäisches Patentamt
European Patent Office
Office européen des brevets

(11) **EP 4 541 400 B1**

(12) **EUROPEAN PATENT SPECIFICATION**

(45) Date of publication and mention
of the grant of the patent:
**08.07.2026 Bulletin 2026/28**

(51) International Patent Classification (IPC):
***A61M 16/04*** *(2006.01)*

(52) Cooperative Patent Classification (CPC):
**A61M 16/047**

(21) Application number: **23203802.6**

(22) Date of filing: **16.10.2023**

(54) **A TRACHEOSTOMA SEALING DEVICE**

TRACHEOSTOMAVERSIEGELUNGSVORRICHTUNG

DISPOSITIF D'OBTURATION DE TRACHÉOSTOMIE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**23.04.2025 Bulletin 2025/17**

(73) Proprietor: **IntraCair ApS**
**8300 Odder (DK)**

(72) Inventors:
• **Juelsgaard Christensen, Karen**
**Odder (DK)**
• **Pedersen, Troels**
**Gilleleje (DK)**

• **Hansen, Peter Werner**
**Birkerød (DK)**
• **Tophøj Heinemann, Signe**
**Hellerup (DK)**
• **Hesseldahl, Mads**
**Frederiksberg (DK)**

(74) Representative: **Valet Patent Services Limited**
**c/o Caya 83713X**
**Am Börstig 5**
**96052 Bamberg (DE)**

(56) References cited:
**WO-A1-2016/001251     WO-A1-94/15657**
**US-B2- 7 556 042**

## Description

### Technical Field

The present disclosure relates generally to a tracheostoma sealing device comprising an inner seal member and an outer anchor, and more particularly relates to a tracheostoma sealing device comprising an inner seal member and an outer anchor, the outer anchor applies a pulling force on the inner seal member such that the pulling force increases as the distance between the inner seal member and the outer anchor increases.

### Background

A tracheostomy tube is a short, curved tube inserted through a surgical hole in a patient's neck and held in place with a tracheostomy tie around the patient's neck. The purpose of a tracheostomy tube is to provide the patient with an airway or to provide the patient with an access that facilitates the suction of secretions from the airway.

Patients have tracheostomy tubes placed for numerous reasons. Once the reason that necessitated the placement of a tracheostomy tube has been resolved, the tracheostomy tube is normally removed. This procedure is known as decannulation. After the patient is decannulated, there remains a hole in the patient's neck which is known as a stoma. Once the patient is decannulated the stoma will usually close on its own in the majority of the patients. This normally occurs in two to three weeks. Surgical closure of the stoma is required for the patients whose stomas do not close on their own.

Currently, when a patient is decannulated, an occlusive or simply a gauze bandage type of arrangement is placed on the patient for days following decannulation. Once the patient leaves the hospital and, in some cases, even within the hospital such as from an Intensive Care Unit to a Regular Care Unit, the usual method of covering the stoma is to use a bandage. The patient is then instructed to cover the stoma with a finger when the patient needs to speak or cough.

This method of covering the stoma with a bandage and using a finger to occlude the stoma in order to talk suffers from a number of disadvantages. First, the patient may not be physically able to occlude the stoma with a finger as needed. Second, in all patients, if the stoma is not covered in a somewhat airtight fashion, air passing in and out of the stoma when the patient attempts to talk causes difficulty with (or even makes it impossible) for the patient to speak. Third, when the patient coughs without covering the stoma, air is forced out of the stoma, and even normal breathing can cause air to pass in and out of the stoma if only covered by a bandage. This air movement through the stoma reduces the chance of the stoma closing on its own without surgery. Fourth, touching the stoma with the patient's fingers to talk or cough greatly increases the chances for infection of the patient's stoma and airway, and may lead to contamination and spreading of infection. Fifthly, in existing products, the pressure over the stoma is not constant and air leaks out during speaking and coughing, the patient thus loses their ability to cough effectively. There is also a risk of Atelectasis (the collapse or closure of alveoli), which can be a consequence of lack of post-expiratory pressure and may affect part or all of a lung resulting in hypoventilation with reduced or absent gas exchange. Atelectasis often correlates with the accumulation of mucus, and it may lead to pneumonia in some cases. Existing products may aim to solve any/all of the above issues by providing a sealing device to seal the stoma. In certain existing products, a sealing element is disposed inside the patient's neck which seals the inner opening of the stoma, and an outer element rests on the skin of the patient which anchors (for example, using a thread) the sealing element in place. However, the sealing element disposed inside the patent's neck is potentially prone to dislodging (e.g. during movement of the patient) such that the seal of the stoma may be reduced. WO2016/001251A1 discloses a tracheostoma sealing device from the prior art.

Accordingly, it is desirable to provide improved devices for sealing a stoma which are less likely to dislodge. Furthermore, other desirable features and characteristics of the present disclosure will become apparent from the subsequent detailed description and the appended claims, taken in conjunction with the accompanying drawings and the foregoing technical field and background.

### Summary

In an aspect, there is provided a tracheostoma sealing device, comprising: an inner seal member for insertion through a tracheostoma of a patient, the inner seal member being configured to seal an inner opening of the tracheostoma; and an outer anchor for placement on an outer side of the tracheostoma, wherein the outer anchor is configured to apply a pulling force on the inner seal member so as to hold the inner seal member against the inner opening of the tracheostoma thereby sealing the inner opening, and wherein the tracheostoma sealing device is configured such that the pulling force increases as the distance between the inner seal member and the outer anchor increases.

In embodiments, the tracheostoma sealing device is configured such that the pulling force increases linearly as the distance between the inner seal member and the outer anchor increases.

In embodiments, the tracheostoma sealing device is configured such that the pulling force increases non-linearly as the distance between the inner seal member and the outer anchor increases.

In embodiments, the tracheostoma sealing device is configured such that a derivative of the pulling force with respect to the distance between the inner seal member and the outer anchor is greater at smaller distances than larger distances.

In embodiments, the tracheostoma sealing device is configured such that a derivative of the pulling force with respect to the distance between the inner seal member and the outer anchor is approximately zero above a maximum distance threshold.

In embodiments, the tracheostoma sealing device comprising a connecting element configured to apply the pulling force on the inner seal member.

In embodiments, the connecting element is configured such that the outer anchor is configured to be able to apply a pushing force and/or rotational force on the inner seal member.

In embodiments, the connecting element comprises a thread connected to the inner seal member, and wherein the thread is configured to apply the pulling force on the inner seal member.

In embodiments, the thread is elastic such that the pulling force increases as the distance between the inner seal member and the outer anchor increases.

In embodiments, the outer anchor comprises a biasing means connected to the thread, the biasing means being configured such that the pulling force increases as the distance between the inner seal member and the outer anchor increases, optionally, wherein the biasing means is one or more springs.

In embodiments, the inner seal member is configured such that as the pulling force increases, a contact surface area of the inner seal member with the patient increases.

In embodiments, the inner seal member is configured such that contact surface area is generally ring-shaped.

In embodiments, the inner seal member and/or the outer anchor are elastic.

In embodiments, the tracheostoma sealing device is configured such that the pulling force is between approximately 0.1N to approximately 30N, optionally, between 10N to 30N.

In embodiments, the tracheostoma sealing device is configured such that the pulling force increases linearly between approximately 0.1N to approximately 30N, optionally, between 10N to 30N.

In embodiments, the tracheostoma sealing device is configured such that inner seal member exerts a maximum pressure of approximately 500 cmH2O to approximately 700 cmH2O, optionally, approximately 600 cmH2O.

## Brief Description of the Drawings

In order to better understand the subject matter that is disclosed herein and to exemplify how it may be carried out in practice, embodiments will now be described, by way of non-limiting example only, with reference to the accompanying drawings, in which:

Fig. 1 is a is a cross-sectional representation of a tracheostoma in a patient;

Fig. 2 is a schematic drawing of a tracheostoma sealing device;

Fig. 3 shows the tracheostoma sealing device of Fig. 2 implanted in a patient;

Fig. 4 is a graph showing how a pulling force F may be varied with a distance between an inner seal member and an outer anchor of the tracheostoma sealing device of Fig. 2;

Fig. 5 is another graph showing how a pulling force F may be varied with a distance between an inner seal member and an outer anchor of the tracheostoma sealing device of Fig. 2;

Fig. 6 is yet another graph showing how a pulling force F may be varied with a distance between an inner seal member and an outer anchor of the tracheostoma sealing device;

Fig. 7 is yet another graph showing how a pulling force F may be varied with a distance between an inner seal member and an outer anchor of the tracheostoma sealing device;

Fig. 8 is another graph showing how a pulling force F may be varied with a distance between an inner seal member and an outer anchor of the tracheostoma sealing device;

Fig. 9 shows a configuration of the outer anchor of the tracheostoma sealing device;

Fig. 10 shows another configuration of the outer anchor of the tracheostoma sealing device;

Fig. 11 shows yet another configuration of the outer anchor of the tracheostoma sealing device; and

Figs. 12A and 12B show the tracheostoma sealing device in two configurations where the distance between an inner seal member and an outer anchor has been changed.

## Detailed Description

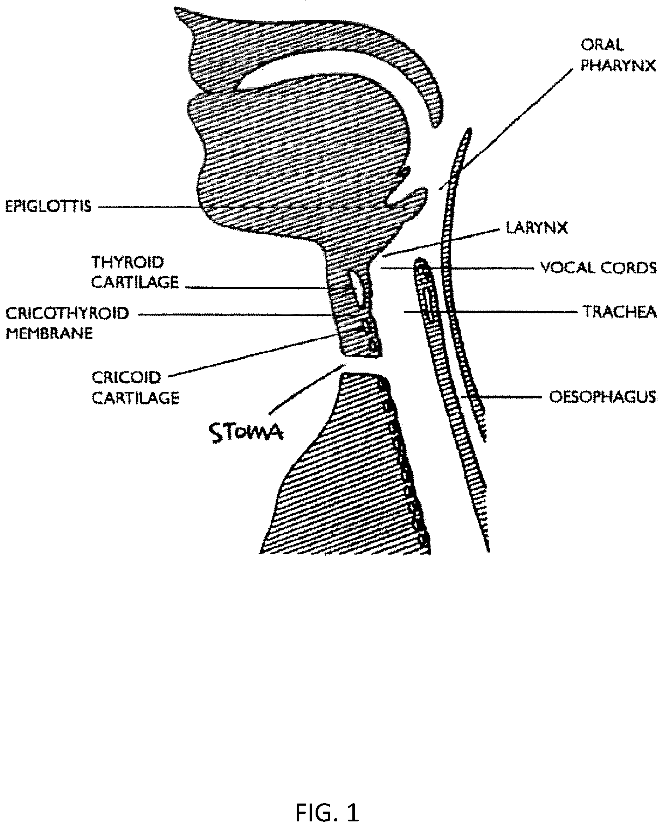

Fig. 1 shows a representation of the anatomy of a patient's neck, showing the location of a surgically-created passage (stoma) through their neck into their trachea. This is called a tracheostoma (stoma). A tracheostoma is created in many different clinical situations and can provide certain benefits to the patient. The surgical procedure to create a tracheostoma is called a tracheotomy. Typically, when such a tracheostoma is created, a tube is inserted through the stoma to allow for the passage of air in a controlled way (this is termed intubation). After a period of time (such as from one week to several months) the tube is removed. The stoma is then allowed to heal and close. As mentioned above, the passage of air through the stoma can slow down the recovery of the patient and may also create other complications and discomforts for the patient. Known devices may be used to seal the stoma. However, since the thickness of the neck may change depending on the position/actions taken by the patient, the sealing device may dislodge. This problem, and others, may be addressed/solved by the following embodiments.

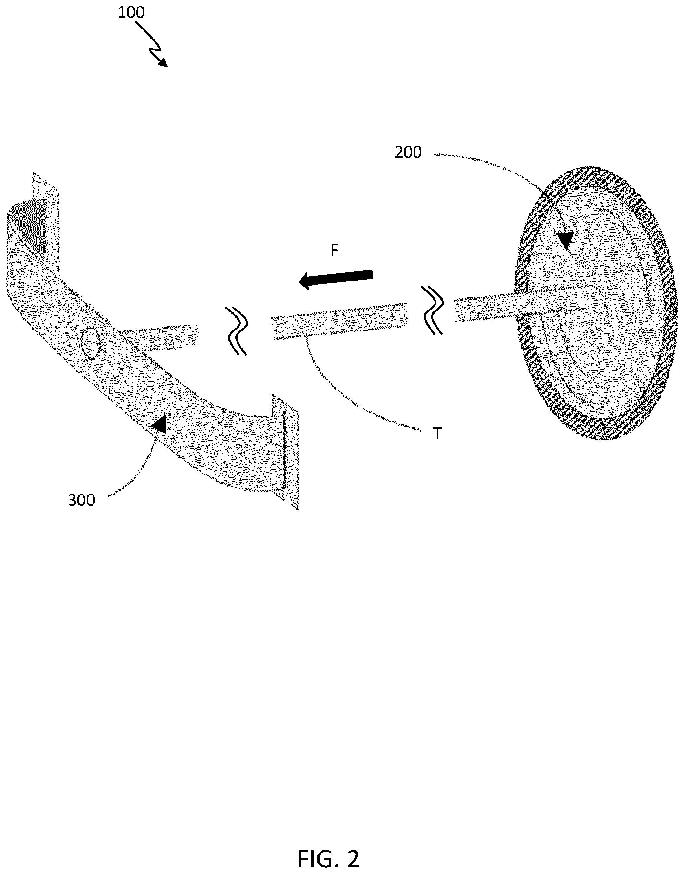

Fig. 2 shows a schematic drawing of a tracheostoma sealing device 100. Generally, the tracheostoma sealing device 100 is for sealing a tracheostoma of a patient. The tracheostoma sealing device 100 may be used once treatment via the tracheostoma is completed and it is intended for the tracheostoma to heal. Healing of the tracheostoma may take a number of days to a few weeks. The tracheostoma sealing device 100 may be configured to be used for such a healing period.

The tracheostoma sealing device 100 comprises an inner seal member 200. The tracheostoma sealing device 100 also comprises an outer anchor 300 having a connecting element in the form of a thread T.

The inner seal member 200 is configured to be placed against an inner opening of the tracheostoma (inside the trachea of the patient) so as to seal in the inner opening of the tracheostoma.

The inner seal member 200 can take many different forms/configurations. In the example shown in Fig. 1, the inner seal member 200 is generally disc shaped. A plane of the disc shape of the inner seal member 200 is configured to placed over the inner opening of the tracheostoma so as to form the seal. The inner seal member 200 may have various structural/functional features so as to improve the seal with the inner opening.

The inner seal member 200 is configured to be inserted through the tracheostoma of the patient (e.g. after the tracheostoma is no longer required for treatment and is intended to heal). For example, the inner seal member 200 is configured to be inserted through a passage no greater than about 9.5 mm. This may be achieved by the inner seal member 200 being able to fold up to a shape which fits within this dimension. For example, the inner seal member 200 may be folded up from a generally planar (disc) shape into a more compressed shape which allows for insertion through the tracheostoma (e.g. via an insertion device such as a sheath). Once inside the trachea, the inner seal member 200 is configured to expand to assume a sealing configuration which allows it to form a seal against the tracheostoma. For a disc-like member, this can be achieved by the use of resilient material which ensures that the disc unfolds into a more planar shape once inserted into the trachea, which is wider than the tracheostoma.

The outer anchor 300 is configured to be placed on an outer side of the tracheostoma (e.g. on the neck of the patient). Generally, the outer anchor 300 acts to hold the inner seal member 200 in place once it has been deployed to seal the inner opening of the tracheostoma. The outer anchor 300 applies a pulling force F on the inner seal member 200 which holds the inner seal member 200 in place over the inner opening of the tracheostoma. To this end, the outer anchor 300 may comprise a connecting element such as, but not limited to, thread T (as shown in Fig. 1) which is connected to the inner seal member 200 (in certain embodiments, the thread T may be integral with the inner seal member 200). The outer anchor 300 via the thread T acts to pull on the inner seal member 200 so as to hold it in place.

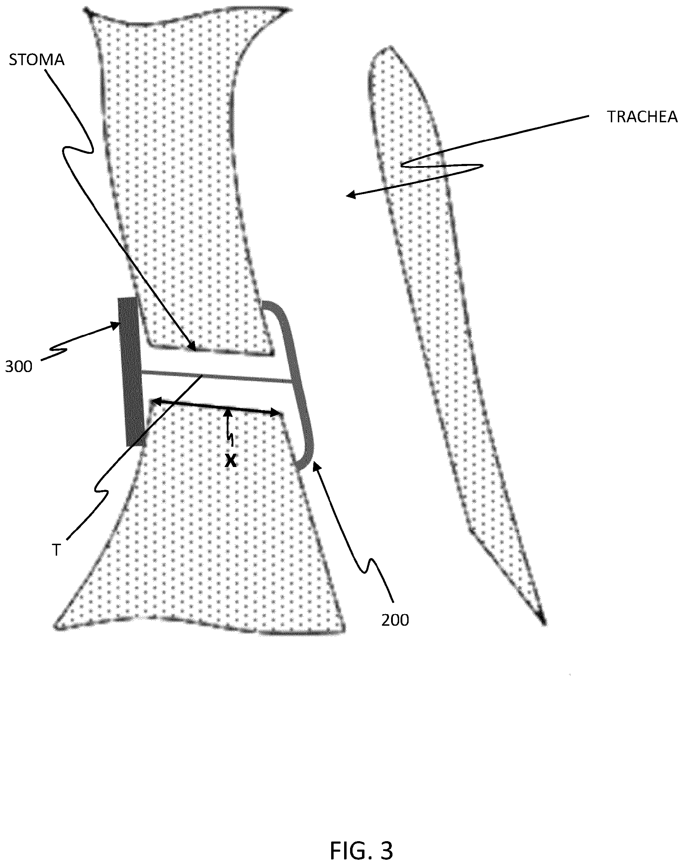

Fig. 3 shows a cross-sectional representation of the tracheostoma sealing device 100 implanted in a patient. The inner seal member 200 is placed in the trachea. The inner seal member 200 is configured to be positioned over the tracheostoma, and specially, the inner opening of the tracheostoma. In such a position, the inner seal member 200 acts to seal the tracheostoma from fluid and air connection to the air outside the patient. To hold the inner seal member 200 in place, the outer anchor 300 is placed on the outside of the patient (on the skin of the neck). The outer anchor 300 may be placed generally opposite the inner seal member 200. The connecting element, in the form of thread T, of the outer anchor 300 passes through the stoma and is configured to pull on the inner seal member 200 to hold it in place.

As discussed in further detail below, the tracheostoma sealing device 100 is configured such that the pulling force F increases as the distance between the inner seal member 200 and the outer anchor 300 increases. Herein, the distance between the inner seal member 200 and the outer anchor 300 may refer to the spacing between the inner seal member 200 and the outer anchor 300 (e.g. as marked by the distance x in Fig. 3). This distance x may generally correspond to the thickness of the patient's neck at the tracheostoma. Other measurements of distance x may be used in accordance with the present disclosure.

The increase in distance between the inner seal member 200 and the outer anchor 300 may be caused by movement of the patient, especially during exercise. In previous devices, any such movement was prone to dislodging the inner seal member such that the tracheostoma is not properly sealed. By the pulling force F increasing as the distance between the inner seal member 200 and the outer anchor 300 increases, the inner seal member 200 is less prone to movement, creating unintended leakage As described below, the pulling force F may be varied in many different ways with respect to the distance between inner seal member 200 and the outer anchor 300. Certain relationships are now described.

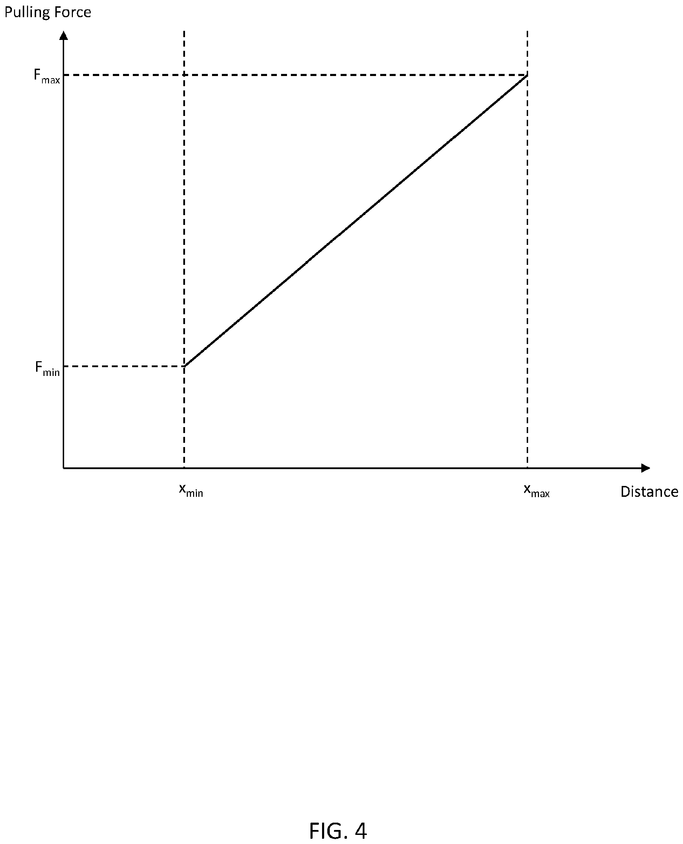

Fig. 4 shows a graph showing an example of how the pulling force F may be varied with distance between the inner seal member 200 and the outer anchor 300. Generally, the pulling force F is varied generally linearly with the distance between the inner seal member 200 and the outer anchor 300. In certain embodiments, for example, as shown in Fig. 4, the relationship holds true between a minimum distance xmin and a maximum distance xmax. The minimum distance and maximum distance may be the normal working range of the tracheostoma sealing device 100 (and optionally may be dependent on the specific patient).

The tracheostoma sealing device 100 is configured such that the pulling force F is varied generally linearly with the distance between the inner seal member 200 and the outer anchor 300. Several different arrangements/configurations of the tracheostoma sealing device 100 may be used to obtain such a varying pulling force.

For example, the outer anchor 300 may comprise a spring which is configured to bias the connecting element, e.g. thread T. In such configurations, as the distance between the inner seal member 200 and the outer anchor 300 increases, the spring acts to apply a (linearly) greater restoring force. The spring may be a spring which generally follows Hooke's law (for example a helical spring).

In other embodiments, the connecting element, e.g., thread T, may be elastic such that it acts as a biasing element. In such configurations, as the distance between the inner seal member 200 and the outer anchor 300 increases, the (elastic) connecting element acts to apply a (linearly) greater restoring force. The connecting element may generally follow Hooke's law, or it may possess other types of characteristics such that the pulling force on the inner seal member 200 increases as the distance between the inner seal member 200 and the outer anchor 300 increases. In some embodiments, the connecting element may be elastic / formed of an elastic material (e.g. polymer / (natural) rubber). For example, the tread T may be an elastic thread, e.g. formed from polymer / (natural) rubber. In other embodiments, the connecting element may comprise a spring / spring-like structure. The spring / spring-like structure may be configured to achieve other elastic properties compared to an elastic pulling thread T.

Depending on either the choice of material or the shape/form of the connecting element, the characteristics of the pulling force with respect to the distance x may follow different kinds of curves.

In some embodiments, the connecting element may have additional/different purposes and/or modes. For example, the connecting element may be elastic and configured to create a pulling force between the inner seal member 200 and the outer anchor 300, but also be stiff when being pushed or rotated such that the outer anchor 300 and the connecting element may be used for folding/unfolding the inner seal member 200 during insertion and removal.

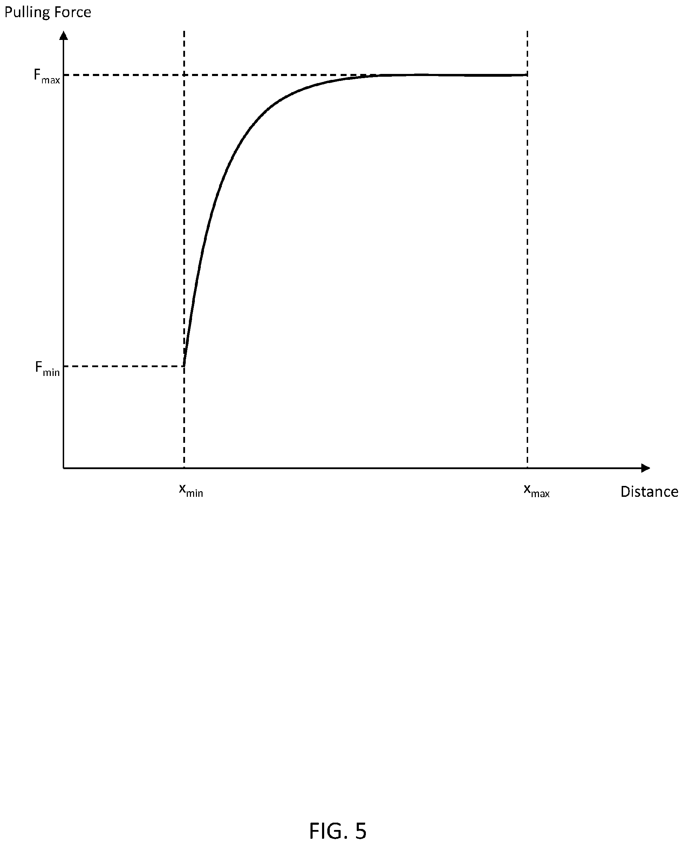

Fig. 5 shows a graph showing an example of how the pulling force F may be varied with distance between the inner seal member 200 and the outer anchor 300. Generally, the pulling force F is varied generally non-linearly with the distance between the inner seal member 200 and the outer anchor 300.

As shown in Fig. 5, the derivative of the pulling force F with respect to the distance between the inner seal member 200 and the outer anchor 300 is the largest at smaller values of the distance (e.g. around xmin). At greater distances ((e.g. around xmax), the derivative decreases, optionally to zero. With such configurations, the pulling force F increases to the greatest extent upon initial increase of the distance such that a large pulling force F can be applied when movement occurs. However, the increases in the pulling force F tapers off to avoid the pulling force F becoming too large (causing pain/discomfort to the patient/damage to the tracheostoma sealing device 100). Several different arrangements/configurations of the tracheostoma sealing device 100 may be used to obtain such a varying pulling force. In some embodiments, such characteristics are obtained by the use of a mechanism comprised in the outer anchor 300. The mechanism may be a physical arrangement of arms that present the above-noted characteristics.

In other embodiments, the above-noted pull force curves may be obtained by use of exotic materials for the connecting element. For example, the connecting element may comprise / consist of super-elastic materials such as memory alloys and/or any other materials in which the properties change once reaching a certain strain or stress.

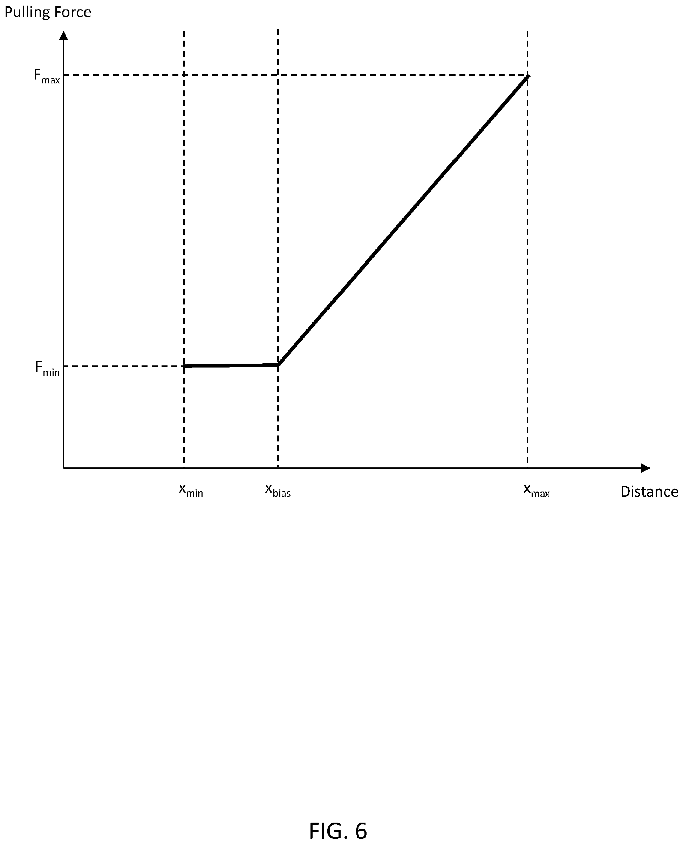

Fig. 6 illustrates yet another example of how the pulling force F may be varied with distance between the inner seal member 200 and the outer anchor 300. In this example, the pulling force F may be constant or almost constant for a certain increase in distance of x (e.g. between xmin and xbias). Once the distance increases above xbias, another characteristic may take over. Such configuration may be useful for ensuring that during small/minor changes in x, the force is not increased (or minimally increased). Only once the distance is x is great enough to risk dislodging, a certain change in characteristic starts to take over.

Accordingly, the tracheostoma sealing device 100 is configured such that a derivative of the pulling force F with respect to the distance x between the inner seal member 200 and the outer anchor 300 is smaller (e.g. approximately zero) at smaller distances x than larger distances x. **In** certain embodiments, the tracheostoma sealing device 100 is configured such that a derivative of the pulling force F with respect to the distance x between the inner seal member 200 and the outer anchor 300 is smaller (e.g. approximately zero) between a first range of distances (e.g. between xmin and xbias) than between a second range of distances ((e.g. greater than xbias). Optionally, the upper value of the first range of distances is lower than the lower value of the second range of distances.

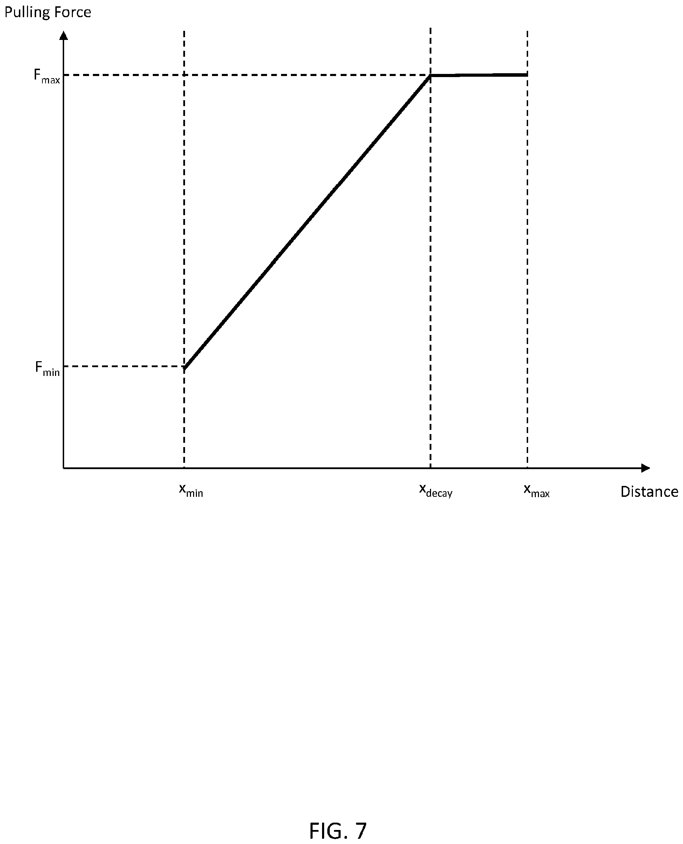

Fig. 7 illustrates yet another example of how the pulling force F may be varied with distance between the inner seal member 200 and the outer anchor 300. In this example, the pulling force F may increase until a certain level of force is reached after which the increase in pulling force F decays to zero. In such a configuration, the tracheostoma sealing device 100 may be configured to prevent providing a pulling force that exceeds an upper threshold Fmax, where Fmax may be a force considered to be traumatic or undesirable for the tracheael mucosal lining.

Accordingly, the tracheostoma sealing device 100 is configured such that a derivative of the pulling force F with respect to the distance x between the inner seal member 200 and the outer anchor 300 is smaller (e.g. approximately zero) at larger distances x than smaller distances x. In certain embodiments, the tracheostoma sealing device 100 is configured such that a derivative of the pulling force F with respect to the distance x between the inner seal member 200 and the outer anchor 300 is smaller (e.g. approximately zero) between a first range of distances (e.g. greater than xdecay) than between a second range of distances (e.g. between xmin and xdecay). Optionally, the upper value of the second range of distances is lower than the lower value of the first range of distances.

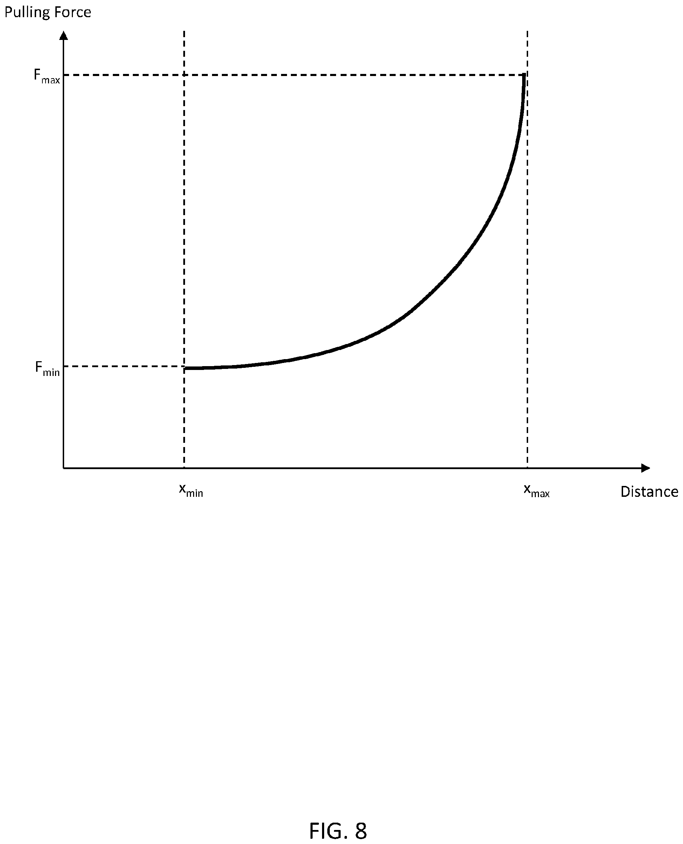

Fig. 8 shows another example of how the pulling force F may be varied with distance between the inner seal member 200 and the outer anchor 300. In this configuration, the pulling force increases increasingly as the distance x increases. Such a configuration may be found useful whenever it is evaluated that minor changes or fluctuations may be present/acceptable during periods of inactivity, and only during more intensive activities (e.g. intense movement during exercise) the pulling force F should increase significantly to avoid dislodging.

Accordingly, the tracheostoma sealing device 100 is configured such that a derivative of the pulling force F with respect to the distance x between the inner seal member 200 and the outer anchor 300 increases as the distance x increases. The tracheostoma sealing device 100 may be configured such that a derivative of the pulling force F with respect to the distance x between the inner seal member 200 and the outer anchor 300 continuously increases as the distance x increases. The tracheostoma sealing device 100 may be configured such that a derivative of the pulling force F with respect to the distance x between the inner seal member 200 and the outer anchor 300 continuously increases as the distance x increases between a range of distances x (e.g. between xmin and xmax).

Even though the exemplary curves described herein include only one or two different curves combined, more advanced combinations of curves may be possible, to achieve more complex and tailored characteristics. For example, in some embodiments, the pulling force F may be constant or almost constant, followed by a stepwise increase in force F to another level of constant or almost constant force. The step representing a steep linear curve (or any other curve) from one level to another level.

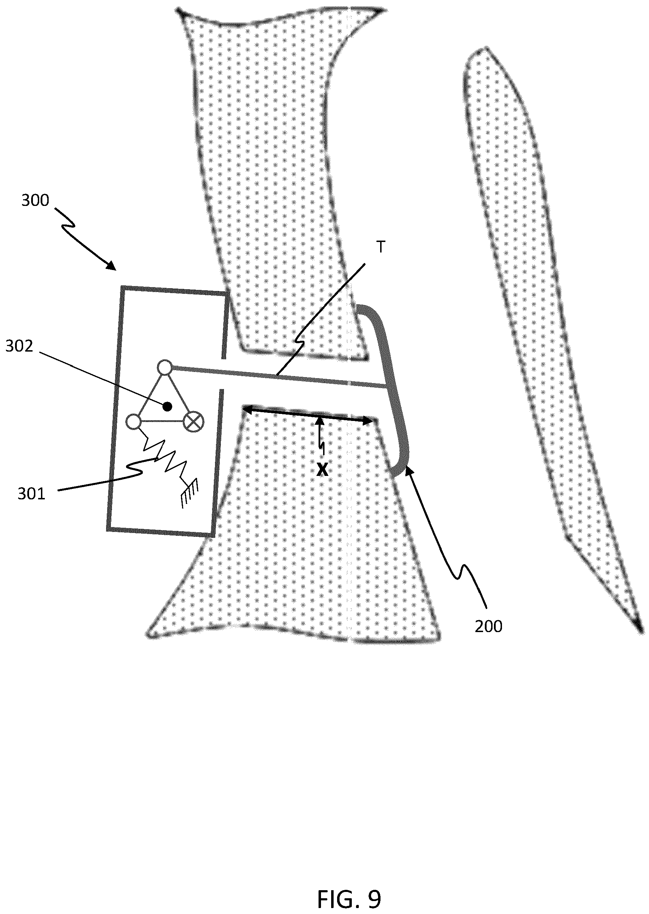

Fig. 9 illustrates an embodiment in which the outer anchor 300 comprises a mechanism configured to convert a spring 301 (e.g. a classic spring following Hooke's Law) into another characteristics by use of a mechanism with a pivoting point 302. Depending on the angles between the spring 301 and the connecting element (e.g. thread T), different conversion or characteristics may be obtained.

It should be understood that even though the illustrated mechanism is a relatively simple type of conversion mechanism, much more complicated mechanisms may be used, changing the characteristics of e.g. a normal spring into other types of pulling force characteristic. These mechanisms may include independent mechanisms changing the characteristics of the pulling force F into one smooth and continuous curve or characteristic. In other mechanisms, the curve may be abrupt/stepwise such that the pulling force F changes differently in different ranges of distances x. This allows different characteristics within each distance x interval.

Accordingly, the outer anchor 300 further comprises a mechanism attached to the connecting element (e.g. thread T). The mechanism may comprise a spring 301 and/or a pivot 302.

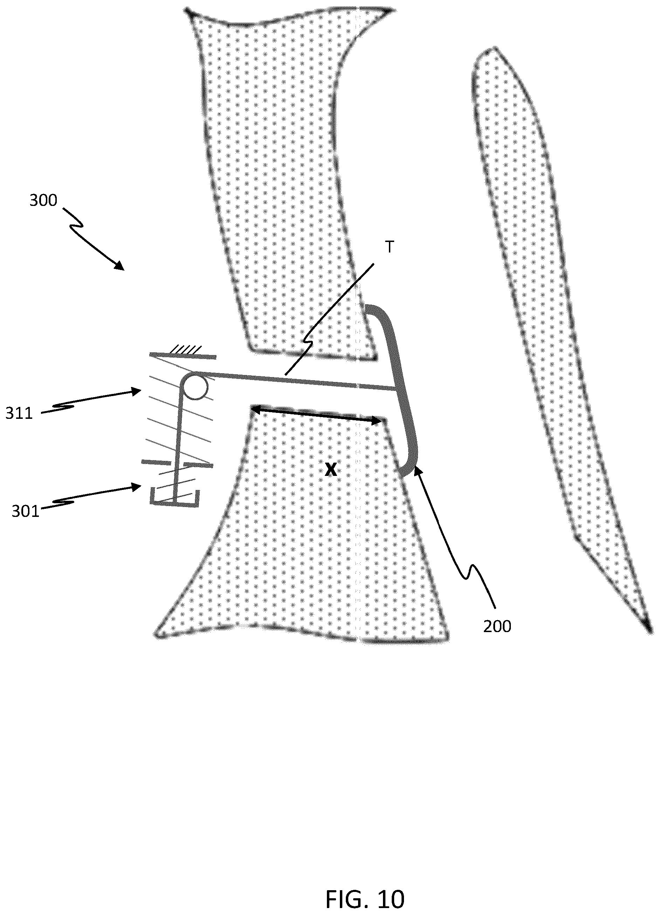

Fig. 10 illustrates another example of a mechanism that may be part of the outer anchor 300. In this embodiment, two springs 301, 311 (e.g. classic springs / non-classic springs) are working in series, e.g. such that the spring 301 is softer than spring 311 or such that spring 301 is a constant force type, allowing the connecting element (e.g. thread T) to stretch a certain length, until the spring 301 is prevented from further compression/elongation, and only the spring 311 may work together with further elongation of distance x. Thus, the tracheostoma sealing device 100 is configured to exert a curve of two different characteristics - for example, the curve illustrated in Fig. 6.

Accordingly, the outer anchor 300 may further comprise a mechanism attached to the connecting element (e.g. thread T). The mechanism may comprise at least two springs (e.g. classic springs / non-classic springs). Optionally, the at least two springs may be connected in series with the connecting element (e.g. thread T).

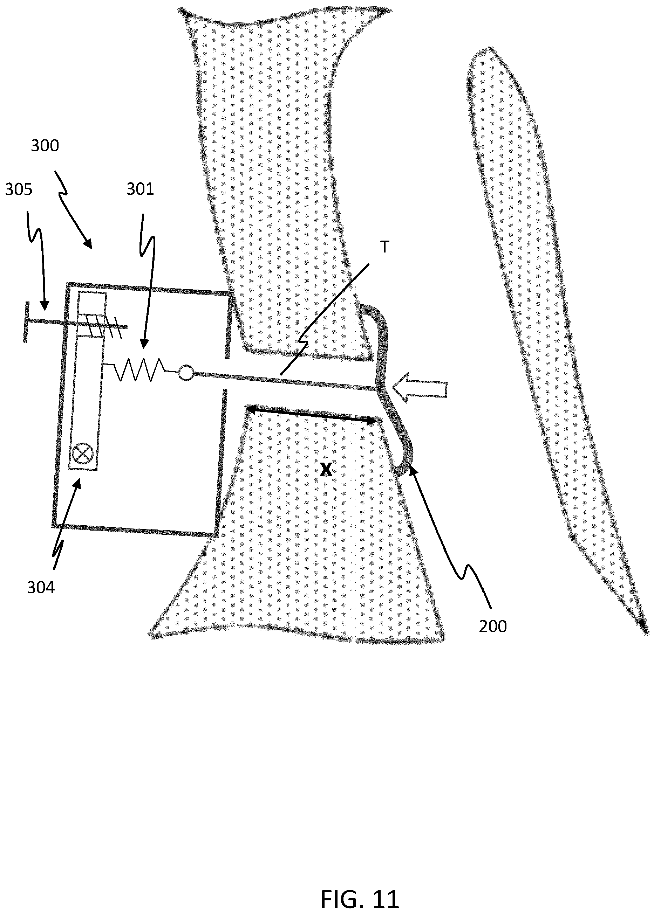

Fig. 11 illustrates yet another example of a mechanism of the outer anchor 300 which comprises an adjustable lever arm 304 attached with a spring 301 (e.g. a classic spring or a non-classic spring), said spring 301 also being connected to the connecting element (e.g. thread T). Further, the inner seal member 200 may be configured to be flexible (e.g. resiliently flexible). Thus, in this example, the tracheostoma sealing device 100 includes a spring 301 and the inner seal member 200 which act as two flexible elements in series, creating desired characteristics. Optionally, the outer anchor 300 may include an adjustment mechanism 304. The adjustment mechanism 304 may be configured to offset the adjustable lever arm 304.

Accordingly, the outer anchor 300 may further comprise a mechanism attached to the connecting element (e.g. thread T). The mechanism may comprise a spring 301. Optionally, the inner seal member 200 is resiliently flexible. Optionally, the spring 301 and the resiliently flexible inner seal member 200 are arranged to act as springs in series.

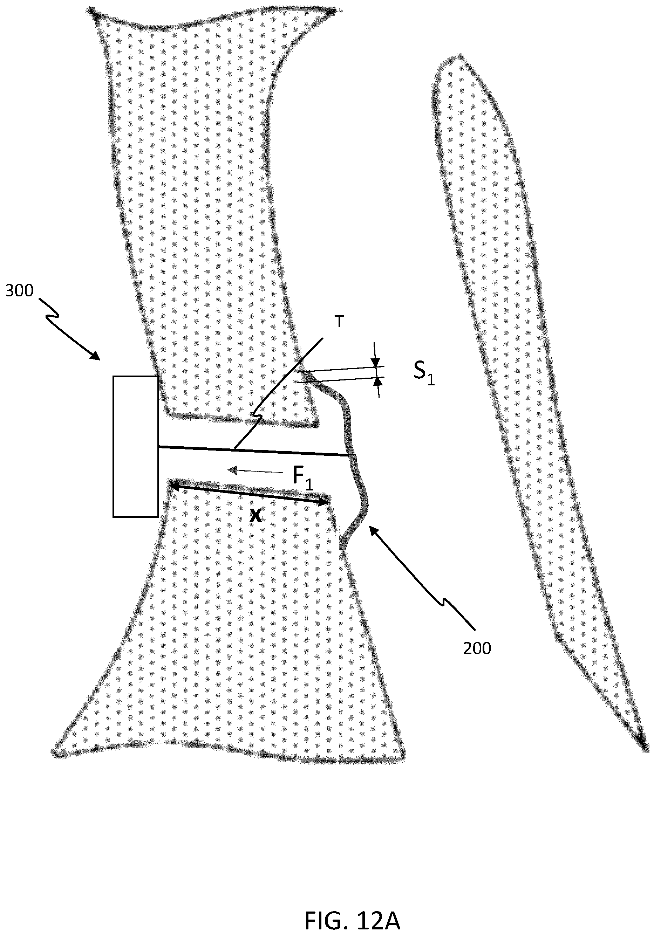

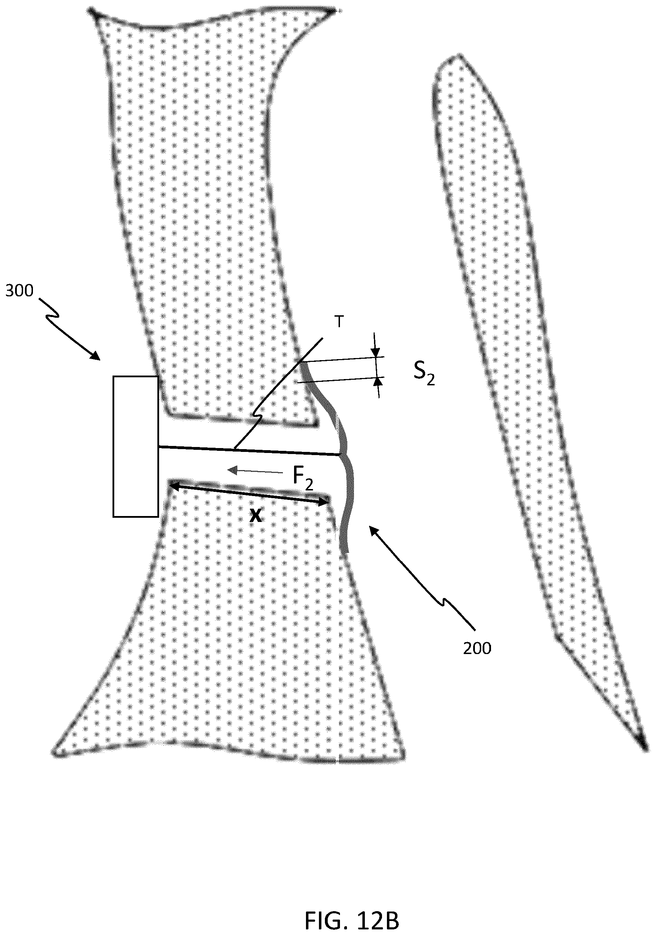

In any of the embodiments disclosed herein, the inner seal member 200 may be flexible, e.g. resiliently flexible. In certain embodiments, the inner seal member 200 may be configured such that a surface area configured to come in contact with trachea increases as the pulling force F is increased. Such configurations may partly compensate for the otherwise greater contact pressure between the tracheal mucosa surface and the seal member. Such configurations may allow for a greater overall anchoring force being applied between the tracheal mucosa and the seal member without greatly increasing the pressure (which reduces chances of discomfort / injury). This is detailed with respect to Figs. 12A and 12B.

As seen in Figs. 12A and 12B, the contact area S of the inner sealing element 200 changes with respect to the pulling force F. As the pulling force F increases, the inner sealing element 200 deforms/flexes and the contact area S against the trachea increases, thus keeping the contact pressure at a desired lower level. For example, the pulling force F may approximately double during increasing of the distance x (e.g. between xmin and xmax). If the inner seal member 200 was a rigid or stiff element with a constant rim of the element resting on the mucosa of the trachea, the contact pressure would also approximately double. The device would be secured against dislodging during movement of the patient, but the contact pressure could exceed a threshold of what is considered safe for avoiding damaging or irritating the tracheal mucosa. The elasticity and flexibility of the inner seal member 200 may provide a mechanism due to which the contact pressure does not increase proportionally with the pulling force F, thereby allowing the pulling force F to increase significantly without increasing the contact pressure proportionally.

Fig. 12A shows a cross-sectional representation of the tracheostoma sealing device 100 implanted in a patient when the distance x (and the pulling force F) is small. In this state, the inner seal member 200 is contacting the tracheal mucosa by an area along the rim of the inner seal member 200. The area may be expressed by using the average contact distance S1. Assuming that the inner sealing member 200 is generally circular with a radius R, the area intersecting with the mucosa may be expressed as:

$$A1 = \pi(R^2 - (R-S1)^2)$$

Given a pulling force F through the connecting element (e.g. thread T), the contact pressure from the inner sealing element towards the mucosa may be expressed:

$$Rho = F/A1$$

Accordingly, if force F doubles and A1 stays the same, the contact pressure is also doubled.

On the other hand, if A is increased with the increased pulling force F, the contact pressure may be kept at a reasonable level. This is shown in Fig. 12B which illustrates a cross-sectional representation of the tracheostoma sealing device 100 implanted in a patient when the distance x (and the pulling force F) is large. In Fig. 12B the same inner seal member 200 is subjected to (resilient) deformation due to a higher pulling force F. During such deformation, the inner seal member 200 may contact the tracheal mucosa to a greater extent. The average distance of the contacting outer rim is defined as S2. S2 is greater than S1 giving a larger contact area A2. Consequently, Rho does not increase to the double value, even though F may be doubled.

In some embodiments, the inner seal member 200 is the most flexible part of the of the tracheostoma sealing device 100. In certain embodiments, the inner seal member 200 is more flexible than the outer anchor 300 and/or the connecting element (e.g. thread T). In certain embodiments, the outer anchor 300 is substantially rigid. In certain embodiments, the connecting element (e.g. thread T) is substantially inextensible.

For the above-described configurations, the inner seal member 200 is designed to both possess flexibility and elasticity, so that during increased distance x between the trachea and the outer skin, the inner seal member 200 may assist in increasing the pulling force F according to a certain profile/characteristic, and simultaneously deform in a way that increases the contact area, which helps to keep the contact pressure low.

In some embodiments, the connecting element (e.g. thread T) may be the most flexible part of the tracheostoma sealing device 100. In certain embodiments, the connecting element is more flexible than the inner seal member 200 and/or the outer anchor 300.

In literature, the recommended maximum contact pressure of the contact area with the tracheal mucosa is 30 cmH2O. However, this maximum value is considered during medium and long term exposure. During very short timeslots a much higher value can be accepted (for example, a value of up to 20 times higher). For example, the inner seal member 200 may be configured to contact the tracheal mucosa with an area of 300 mm2 during resting of the patient, would allow a pulling force of:

$$F = p \times A = 2.94 \text{ kpa} * 300 \text{ mm2} = 0.8 \text{ N}$$

During sudden movements, the pulling force could be allowed twenty times higher, which in the example would be 16 N. During deformation of the inner seal member 200, the area contacting the mucosa may be even further increased, or the sealing device may have a bigger size, which could bring the short-term maximum pulling force up to 30 N without risk of damaging the trachea mucosa.

Accordingly, with the arrangements disclosed herein, the maximum pulling force F may therefore be 30 N during elongation of the distance x. Thus, this may be considered as either the maximum value expected to be reached with the maximum value of x, or it may be the upper limit of pulling force F, before the characteristics level out and become almost constant (e.g. the derivative of the pulling force F with respect to the distance between the inner seal member 200 and the outer anchor 300 is approximately zero).

In other embodiments, the characteristics of the pulling force F during normal modes may have a lower slope or be substantially constant, increasing the pulling force only marginally during the increase of distance x. This may be due to the fact that the characteristics of the device is desired to behave in such a way that even though the distance x may increase somewhat, the pulling force is only slightly increased. For some embodiments, the device may be configured such that the force F may increase by less than 1 N per cm of increase of distance x.

The biasing - or base pulling force F (e.g. at xmin) - may be applied by a practitioner, during installation, and may have a level of between 0.1 N and 10 N depending on the type of contact between the inner seal member 200 and the trachea. In some embodiments of the inner seal member 200, the radius and area of the contact with the trachea may be small, and limited. Thus, the base pulling force F may be very low. In other embodiments, the pulling force F may have a base of 10N, as the inner sealing member 200 may have a large surface, very adaptable to the trachea, thus reducing the contact pressure significantly.

Throughout this disclosure, reference to an elastic thread (or any other elastic element) may refer to a thread/element which is able to resume its normal length/shape spontaneously after being stretched past its equilibrium length, as would be understood by a skilled person. For example, elastic threads / any other elastic element may comprise / consist of any combination of: elastin, rubber, nylon, lycra, silicon, and polyester.

Throughout this disclosure, the pulling force may be characterised by any combination of the pulling forces described herein. For example, the pulling force may be characterised by a constant force interval combined with a linearly increasing interval. In another example, a number of linearly increasing intervals may be combined with different derivates of the pulling force F with respect to the distance between the inner seal member 200 and the outer anchor 300.

While at least one exemplary embodiment has been presented in the foregoing detailed description, it should be appreciated that a vast number of variations exist.

It should also be appreciated that the exemplary embodiments are only examples, and are not intended to limit the scope, applicability, or configuration of the disclosure in any way. Rather, the foregoing detailed description will provide those skilled in the art with a convenient road map for implementing the exemplary embodiment or exemplary embodiments. It should be understood that various changes can be made in the function and arrangement of elements without departing from the scope of the disclosure as set forth in the appended claims.

## Claims

1. A tracheostoma sealing device (100), comprising:

   an inner seal member (200) for insertion through a tracheostoma of a patient, the inner seal member being configured to seal an inner opening of the tracheostoma; and
   an outer anchor (300) for placement on an outer side of the tracheostoma,
   wherein the outer anchor is configured to apply a pulling force on the inner seal member so as to hold the inner seal member against the inner opening of the tracheostoma thereby sealing the inner opening, and
   **characterised in that** the tracheostoma sealing device is configured such that the pulling force increases as the distance between the inner seal member and the outer anchor increases.

2. The tracheostoma sealing device of Claim 1, wherein the tracheostoma sealing device is configured such that the pulling force increases linearly as the distance between the inner seal member and the outer anchor increases.

3. The tracheostoma sealing device of Claim 1, wherein the tracheostoma sealing device is configured such that the pulling force increases non-linearly as the distance between the inner seal member and the outer anchor increases.

4. The tracheostoma sealing device of Claim 3, wherein the tracheostoma sealing device is configured such that a derivative of the pulling force with respect to the distance between the inner seal member and the outer anchor is greater at smaller distances than larger distances.

5. The tracheostoma sealing device of any preceding claim, wherein the tracheostoma sealing device is configured such that a derivative of the pulling force with respect to the distance between the inner seal member and the outer anchor is approximately zero above a maximum distance threshold.

6. The tracheostoma sealing device of any preceding claim, comprising a connecting element configured to apply the pulling force on the inner seal member.

7. The tracheostoma sealing device of Claim 6, wherein the connecting element is configured such that the outer anchor is configured to be able to apply a pushing force and/or rotational force on the inner seal member .

8. The tracheostoma sealing device of Claim 6, wherein the connecting element comprises a thread connected to the inner seal member, and wherein the thread is configured to apply the pulling force on the inner seal member.

9. The tracheostoma sealing device of Claim 8, wherein the thread is elastic such that the pulling force increases as the distance between the inner seal member and the outer anchor increases .

10. The tracheostoma sealing device of Claim 8 or 9, wherein the outer anchor comprises a biasing means connected to the thread, the biasing means being configured such that the pulling force increases as the distance between the inner seal member and the outer anchor increases, optionally, wherein the biasing means is one or more springs .

11. The tracheostoma sealing device of any preceding claim, wherein the inner seal member is configured such that as the pulling force increases, a contact surface area of the inner seal member with the patient increases.

12. The tracheostoma sealing device of Claim 11, wherein the inner seal member is configured such that contact surface area is generally ring-shaped.

13. The tracheostoma sealing device of any preceding claim, wherein the inner seal member and/or the outer anchor are elastic.

14. The tracheostoma sealing device of any preceding claim, wherein the tracheostoma sealing device is configured such that the pulling force is between approximately 0.1N to approximately 30N, optionally, between 10N to 30N, and optionally, wherein the tracheostoma sealing device is configured such that the pulling force increases linearly between approximately 0.1N to approximately 30N, optionally, between 10N to 30N.

15. The tracheostoma sealing device of any preceding claim, wherein the tracheostoma sealing device is configured such that inner seal member exerts a maximum pressure of approximately 500 cmH2O to approximately 700 cmH2O, optionally, approximately 600 cmH2O.

**Patentansprüche**

1. Tracheostoma-Abdichtungsvorrichtung (100), umfassend:

   ein inneres Abdichtungselement (200) zum Einfügen durch ein Tracheostoma eines Patienten, wobei das innere Abdichtungselement konfiguriert ist, um eine innere Öffnung des Tracheostomas zu abdichten; und
   einen äußeren Anker (300) zum Anbringen an einer äußeren Seite des Tracheostomas,
   wobei der äußere Anker konfiguriert ist, um eine Zugkraft auf das innere Abdichtungselement auszuüben, um das innere Abdichtungselement gegen die innere Öffnung des Tracheostomas zu halten, wodurch die innere Öffnung abgedichtet wird, und
   **dadurch gekennzeichnet, dass** die Tracheostoma-Abdichtungsvorrichtung derart konfiguriert ist, dass die Zugkraft erhöht wird, wenn die Distanz zwischen dem inneren Abdichtungselement und dem äußeren Anker erhöht wird.

2. Tracheostoma-Abdichtungsvorrichtung nach Anspruch 1, wobei die Tracheostomaabdichtungsvorrichtung derart konfiguriert ist, dass die Zugkraft erhöht wird, wenn die Distanz zwischen dem inneren Abdichtungselement und dem äußeren Anker linear erhöht wird.

3. Tracheostoma-Abdichtungsvorrichtung nach Anspruch 1, wobei die Tracheostomaabdichtungsvorrichtung derart konfiguriert ist, dass die Zugkraft erhöht wird, wenn die Distanz zwischen dem inneren Abdichtungselement und dem äußeren Anker nicht linear erhöht wird.

4. Tracheostoma-Abdichtungsvorrichtung nach Anspruch 3, wobei die Tracheostoma-Abdichtungsvorrichtung derart konfiguriert ist, dass eine Ableitung der Zugkraft im Hinblick auf die Distanz zwischen dem inneren Abdichtungselement und dem äußeren Anker bei kleineren Distanzen größer ist als bei größeren Distanzen.

5. Tracheostoma-Abdichtungsvorrichtung nach Anspruch 1, wobei die Tracheostoma-Abdichtungsvorrichtung derart konfiguriert ist, dass eine Ableitung der Zugkraft im Hinblick auf die Distanz zwischen dem inneren Abdichtungselement und dem äußeren Anker annähernd null ist, wenn der maximale Distanzschwellenwert überschritten wird.

6. Tracheostoma-Abdichtungsvorrichtung nach einem der vorstehenden Ansprüche, umfassend ein Verbindungsbauteil, das konfiguriert ist, um die Zugkraft auf das innere Abdichtungselement auszuüben.

7. Tracheostoma-Abdichtungsvorrichtung nach Anspruch 6, wobei das Verbindungsbauteil derart konfiguriert ist, dass der äußere Anker so konfiguriert ist, dass er eine Druckkraft und/oder eine Drehkraft auf das innere Abdichtungselement ausüben kann.

8. Tracheostoma-Abdichtungsvorrichtung nach Anspruch 6, wobei das Verbindungsbauteil ein mit dem inneren Abdichtungselement verbundenes Gewinde umfasst und wobei das Gewinde konfiguriert ist, um die Zugkraft auf das innere Abdichtungselement auszuüben.

9. Tracheostoma-Abdichtungsvorrichtung nach Anspruch 8, wobei der Faden derart elastisch ist, dass die Zugkraft erhöht wird, wenn die Distanz zwischen dem inneren Abdichtungselement und dem äußeren Anker erhöht wird.

10. Tracheostoma-Abdichtungsvorrichtung nach Anspruch 8 oder 9, wobei der äußere Anker eine mit dem Faden verbundene Vorspannvorrichtung umfasst, die derart konfiguriert ist, dass die Zugkraft erhöht wird, wenn die Distanz zwischen dem inneren Abdichtungselement und dem äußeren Anker erhöht wird, wobei die Vorspannvorrichtung optional eine oder mehrere Federn ist.

11. Tracheostoma-Abdichtungsvorrichtung nach einem der vorstehenden Ansprüche, wobei das innere Abdichtungselement derart konfiguriert ist, dass sich die Kontaktfläche des inneren Abdichtungselements mit dem Patienten erhöht, wenn die Zugkraft erhöht wird.

12. Tracheostoma-Abdichtungsvorrichtung nach Anspruch 11, wobei das innere Abdichtungselement derart konfiguriert ist, dass die Kontaktfläche im Allgemeinen ringförmig ist.

13. Tracheostoma-Abdichtungsvorrichtung nach einem der vorstehenden Ansprüche, wobei das innere Abdichtungselement und/oder die äußere Verankerung elastisch sind.

14. Tracheostoma-Abdichtungsvorrichtung nach einem der vorstehenden Ansprüche, wobei die Tracheostoma-Abdichtungsvorrichtung derart konfiguriert ist, dass die Zugkraft zwischen etwa 0,1 N und etwa 30 N, optional zwischen 10 N und 30 N, liegt, und optional wobei die Tracheostoma-Abdichtungsvorrichtung derart konfiguriert ist, dass die Zugkraft linear zwischen etwa 0,1 N und etwa 30 N erhöht wird, optional zwischen 10 N und 30 N.

15. Tracheostoma-Abdichtungsvorrichtung nach einem der vorstehenden Ansprüche, wobei die Tracheostoma-Abdichtungsvorrichtung derart konfiguriert ist, dass das innere Abdichtungselement einen maximalen Druck von etwa 500 $cmH_2O$ bis etwa 700 $cmH_2O$, optional etwa 600 $cmH_2O$, ausübt.

**Revendications**

1. Dispositif d'étanchéité de trachéostomie (100), comprenant :

un élément d'étanchéité interne (200) destiné à être inséré à travers une trachéostomie d'un patient, l'élément d'étanchéité interne étant configuré pour sceller une ouverture interne de la trachéostomie ; et
un ancrage externe (300) destiné à être placé sur un côté externe de la trachéostomie,
dans lequel l'ancrage externe est configuré pour appliquer une force de traction sur l'élément d'étanchéité interne de façon à maintenir l'élément d'étanchéité interne contre l'ouverture interne de la trachéostomie, scellant ainsi l'ouverture interne, et
**caractérisé en ce que** le dispositif d'étanchéité de trachéostomie est configuré de telle sorte que la force de traction augmente au fur et à mesure que la distance entre l'élément d'étanchéité interne et l'ancrage externe augmente.

2. Dispositif d'étanchéité de trachéostomie selon la revendication 1, dans lequel le dispositif d'étanchéité de trachéostomie est configuré de telle sorte que la force de traction augmente de manière linéaire au fur et à mesure que la distance entre l'élément d'étanchéité interne et l'ancrage externe augmente.

3. Dispositif d'étanchéité de trachéostomie selon la revendication 1, dans lequel le dispositif d'étanchéité de trachéostomie est configuré de telle sorte que la force de traction augmente de manière non-linéaire au fur et à mesure que la distance entre l'élément d'étanchéité interne et l'ancrage externe augmente.

4. Dispositif d'étanchéité de trachéostomie selon la revendication 3, dans lequel le dispositif d'étanchéité de trachéostomie est configuré de telle sorte qu'une dérivée de la force de traction par rapport à la distance entre l'élément d'étanchéité interne et l'ancrage externe est supérieure aux distances plus petites qu'aux distances plus grandes.

5. Dispositif d'étanchéité de trachéostomie selon l'une des revendications précédentes, dans lequel le dispositif d'étanchéité de trachéostomie est configuré de telle sorte qu'une dérivée de la force de traction par rapport à la distance entre l'élément d'étanchéité interne et l'ancrage externe est environ nulle au-dessus d'une distance seuil maximale.

6. Dispositif d'étanchéité de trachéostomie selon l'une des revendications précédentes, comprenant un élément de liaison configuré pour appliquer la force de traction sur l'élément d'étanchéité interne.

7. Dispositif d'étanchéité de trachéostomie selon la revendication 6, dans lequel l'élément de liaison est configuré de telle sorte que l'ancrage externe est configuré de façon à pouvoir appliquer une force de poussée et/ou une force de rotation sur l'élément d'étanchéité interne.

8. Dispositif d'étanchéité de trachéostomie selon la revendication 6, dans lequel l'élément de liaison comprend un filetage relié à l'élément d'étanchéité interne, et dans lequel le filetage est configuré pour appliquer la force de traction sur l'élément d'étanchéité interne.

9. Dispositif d'étanchéité de trachéostomie selon la revendication 8, dans lequel le filetage est élastique de telle sorte que la force de traction augmente au fur et à mesure que la distance entre l'élément d'étanchéité interne et l'ancrage externe augmente.

10. Dispositif d'étanchéité de trachéostomie selon la revendication 8 ou 9, dans lequel l'ancrage externe comprend un moyen de rappel relié au filetage, le moyen de rappel étant configuré de telle sorte que la force de traction augmente au fur et à mesure que la distance entre l'élément d'étanchéité interne et l'ancrage externe augmente, éventuellement, dans lequel le moyen de rappel est un ou plusieurs ressort(s).

11. Dispositif d'étanchéité de trachéostomie selon l'une des revendications précédentes, dans lequel l'élément d'étanchéité interne est configuré de telle sorte qu'au fur et à mesure que la force de traction augmente, une surface de contact de l'élément d'étanchéité interne avec le patient augmente.

12. Dispositif d'étanchéité de trachéostomie selon la revendication 11, dans lequel l'élément d'étanchéité interne est configuré de telle sorte que la surface de contact est globalement en forme d'anneau.

13. Dispositif d'étanchéité de trachéostomie selon l'une des revendications précédentes, dans lequel l'élément d'étanchéité interne et/ou l'ancrage externe sont élastiques.

14. Dispositif d'étanchéité de trachéostomie selon l'une des revendications précédentes, dans lequel le dispositif d'étanchéité de trachéostomie est configuré de telle sorte que la force de traction est comprise entre environ 0,1 N et environ 30 N, éventuellement, entre 10 N et 30 N, et éventuellement, dans lequel le dispositif d'étanchéité de trachéostomie est configuré de telle sorte que la force de traction augmente de manière linéaire entre environ 0,1 N et environ 30 N, éventuellement, entre 10 N et 30 N.

15. Dispositif d'étanchéité de trachéostomie selon l'une des revendications précédentes, dans lequel le dispositif d'étanchéité de trachéostomie est configuré de telle sorte que l'élément d'étanchéité interne exerce une pression maximale d'environ 500 cmH2O à environ 700 cmH2O, éventuellement, d'environ 600 cmH2O.

ORAL
PHARYNX

EPIGLOTTIS

THYROID
CARTILAGE

CRICOTHYROID
MEMBRANE

CRICOID
CARTILAGE

STOMA

LARYNX

VOCAL CORDS

TRACHEA

OESOPHAGUS

FIG. 1

FIG. 2

FIG. 3

Pulling Force

FIG. 4

FIG. 5

Pulling Force

$F_{max}$

$F_{min}$

$x_{min}$  $x_{bias}$  $x_{max}$  Distance

FIG. 6

Pulling Force

$F_{max}$

$F_{min}$

$x_{min}$          $x_{decay}$     $x_{max}$          Distance

FIG. 7

Pulling Force

FIG. 8

FIG. 9

FIG. 10

FIG. 11

FIG. 12A

FIG. 12B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016001251 A1